# EUROPEAN PATENT SPECIFICATION

(11) **EP 4 194 051 B1**
(45) Date of publication and mention of the grant of the patent: **10.07.2024**
(21) Application number: 21213074.4
(22) Date of filing: 08.12.2021
(51) Int. Cl.: A61N 1/372

(54) **IMPLANT ARRANGEMENT ENABLING VIRTUAL PROGRAMMING OF AN IMPLANT**
IMPLANTATANORDNUNG ZUM ERMÖGLICHEN VON VIRTUELLER PROGRAMMIERUNG EINES IMPLANTATS
AGENCEMENT D'IMPLANT PERMETTANT LA PROGRAMMATION VIRTUELLE D'UN IMPLANT

(43) Date of publication of application: 14.06.2023
(73) Proprietor: BIOTRONIK SE & Co. KG, 12359 Berlin (DE)
(72) Inventor: Thomas, Dörr, 12437 Berlin (DE); Muessig, Dirk, West Linn, 97068 (US)
(74) Representative: Biotronik Corporate Services SE

(56) References cited:
- US-A1- 2009 281 598
- US-A1- 2012 101 544
- US-A1- 2020 030 618
- US-A1- 2020 398 062
- US-B2- 11 083 372
- US-B2- 6 564 104

## Description

The present invention relates to an implant arrangement according to claim 1 and to a method of post-processing data on at least one diagnostic parameter of a human or animal patient with an implant arrangement according to claim 14

The remote access of devices is generally desirable. However, in case of implantable medical devices, particular care must be taken in order to avoid any unauthorized access to the implantable medical device in order to guarantee safe functioning of the implantable medical device.

There are implantable cardiac rhythm monitors on the market that can be at least partially re-programmed via a Bluetooth Low Energy (BLE) data channel from a remote location.

Such reprogramming results in an amendment of programming settings in the implantable medical device. Therefore, a data transfer track from a remote monitoring system to the implantable medical device to be re-programmed needs to be secured in a particularly elaborate manner. The most demanding difficulty is the communication track between the implantable medical device and an external patient communicator such as a common smartphone. Particularly secured communication protocols are necessary for a safe data transmission. These communication protocols need to be updated in case of discovering a potential cyber security weakness. Consequently, such systems that allow a remote access to an implantable medical device require continuous and long-lasting maintenance.

US 2012/101544 A1 generally pertains to implantable medical devices and more particularly to a non-programmable activation device that switches modes of operation of an implantable medical device and methods for using the activation device. A method is provided for changing settings of an implantable medical device (IMD) prior to a medical procedure.

US 11,083,372 B2 relates to implantable medical devices such as pacemakers, defibrillators, and cardiac resynchronization therapy devices. A system comprises a medical device configured to: sense a non-stimulating sync signal; sense physiological parameter measurements to generate a physiological signal; and in response to sensing the sync signal, generate a sync-coupled physiological signal.

US 6,564,104 B2 pertains to an information network for remotely directing and managing patient device data retrieval and device instruction updates. Provided is a system for a clinician or other person or device to verify communication link bandwidth prior to and during real time data communications, greatly increasing the information security and reliability of the comprehensive system for managing the IMDs.

US 2009/0281598 A1 relates to methods for remotely programming an implantable medical device (IMD) and is directed toward providing a medical device system and method for remotely programming an IMD, which includes safety and security features for preventing unauthorized, unsecure, or unsafe programming of the IMD.

US 2020/0030618 A1 relates to cardiac therapy delivery by implantable medical devices and is directed to techniques for controlling the delivery of cardiac therapy (e.g., cardiac pacing) by an IMD to a heart of a patient. Such techniques may include receiving a patient request from an application running on a patient personal device (e.g., a smartphone or a tablet), and determining a requested value of a therapy parameter for cardiac pacing based on the request.

US 2020/0398062 A1 is directed to a system, method and a network architecture for facilitating remote care therapy via secure communication channels between clinicians and patients having one or more implantable medical devices (IMDs), wherein certain unique information retrieved from an IMD is used in association with an encryption key infrastructure system for establishing trusted relationships between a clinician device, a patient's device and the patient's IMD.

It is an object of the present invention to provide an implant arrangement that comprises an implantable medical device having a diagnostic function, wherein the data gathered by this implantable medical device can be analyzed in many different ways to address different medical questions, while the risk of an unauthorized access to the implantable medical device (such as by a cyber-attack) is significantly reduced or even completely avoided. This object is achieved with an implant arrangement having the features of claim 1. Such an implant arrangement comprises an active implantable medical device and a remote data-monitoring device.

The term "active implantable medical device" is well known to a person skilled in the art. An "active medical device" is typically defined to be any medical device relying for its functioning on a source of electrical energy or any source of power other than that directly generated by the human body or gravity.

An "active implantable medical device" is typically defined to be any active medical device which is intended to be totally or partially introduced, surgically or medically, into the human body or by medical intervention into a natural orifice, and which is intended to remain after the procedure.

The active implantable medical device comprises a sensing unit for sensing at least one diagnostic parameter of a human or animal patient once the device is implanted into the body of the patient. The active implantable medical device further comprises a data transfer interface for transferring data on the at least one diagnostic parameter sensed by the sensing unit to the remote data-monitoring device.

The remote data-monitoring device is configured to apply least one post-processing function on the data received from the active implantable medical device. Furthermore, the remote data-monitoring device is configured to allow an adjustment of at least one configuration parameter of the post-processing function. This adjustment takes place dependent on a user input received by the remote data-monitoring device. Thus, the user can actively adjust the at least one configuration parameter by an input.

The at least one configuration parameter alters an effect of the post-processing function on the data received from the active implantable medical device.

Thus, the presently claimed implant arrangement does not require a remote access to the active implantable medical device in order to achieve a physical reprogramming of the active implantable medical device. Rather, the presently claimed implant arrangement allows a virtual reprogramming of the active implantable medical device, namely, by adjusting the post-processing function used for analyzing the data obtained by the active implantable medical device and transferred to the remote data-monitoring device.

Due to this virtual programming of the active implantable medical device, no access to the active implantable medical device itself is necessary. Rather, by virtually manipulating the data transferred from the active implantable medical device to the remote data-monitoring device, only an access to the remote data-monitoring device is necessary. Such an access, however, can be granted with a much lower cyber security level since any manipulations performed on the data being present on the remote data-monitoring device will not have an impact on the functionality of the active implantable medical device. Thus, even if an unauthorized access to the remote data-monitoring device would be successful, this would have no impact on the functionality and safety of the active implantable medical device itself.

Consequently, the active implantable medical device of the presently claimed implant arrangement does not require a communication unit to an external device that is secured in a particularly elaborate manner. Rather, the standard levels of cyber security can be applied to the active implantable medical device. This facilitates manufacturing of the active implantable medical device and reduces costs and maintenance work. Furthermore, the lifetime of such an active implantable medical device is increased since it will no longer depend on the continued support of a specific data communication protocol or interface.

In an embodiment, the sensing unit is configured to sense the at least one diagnostic parameter with the lowest possible sensing threshold. Thus, the sensing unit senses the diagnostic parameter with the highest sensitivity. In doing so, the amount of sensed data is increased with respect to sensing modes in which a higher sensing threshold is applied. This results in an increased data volume to be handled by the active implantable medical device. However, the internal data handling of the active implantable medical device does not require a significant amount of energy. Furthermore, the energy demand of the data transfer interface is also not significantly increased in case of transmitting a higher amount of data. To give an example, a data transfer according to the Bluetooth Low Energy (BLE) standard requires the most energy for establishing a connection. The data transfer itself does not require a significant amount of energy. Therefore, the approach taken in this embodiment, namely, gathering as many data as possible, does not negatively influence the lifetime of the active implantable medical device in a significant way since its energy consumption is not significantly increased.

In an embodiment, the data transfer interface (which can also be denoted as data communication unit) serves for directly or indirectly transferring data to the remote data-monitoring device in a wireless manner. All standard data transmission protocols or specifications are appropriate for at least a section of such a wireless data communication. Examples of standard data transmission protocols or specifications are the Medical Device Radiocommunications Service (MICS), the Bluetooth Low Energy (BLE) protocol and the Zigbee specification.

In an embodiment, the data transfer interface is configured to allow exclusively a data transfer away from the active implantable medical device (i.e., out of the active implantable medical device), but not towards the active implantable medical device. By such a one-way configuration of the data transfer interface, any cyber-attacks directed to the active implantable medical device and addressing the data transfer interface are efficiently circumvented.

In some instances, it may be desired to allow an access to the active implantable medical device by a programming device addressing not the data transfer interface, but a separate programming data transfer interface using different communication protocols and a different authentication than the data transfer interface. In such a case, it is still possible to adjust physical settings of the active implantable medical device by the programming device located nearby the active implantable medical device.

In an embodiment, the whole active implantable medical device is configured to allow exclusively a data transfer away from the active implantable medical device, but not towards the active implantable medical device. In this embodiment, also an access by a programming device to the settings of the active implantable medical device is not possible. This embodiment is particularly appropriate in case of a purely diagnostic implant that can, consequently, be operated in a particularly secure manner.

A possible way of implementing the precedingly explained embodiment is a design of the active implantable medical device in which the data transfer interface is the only data transfer interface of the active implantable medical device. Therefore, this design is implemented in an embodiment of the implant arrangement.

In an embodiment, the patient is able to receive data from the implantable medical device with an app on a smartphone (via the data transfer interface). In an embodiment, the patient can furthermore use the smartphone to further transfer the data to the remote data-monitoring device. Even if a physician manipulates the data on the remote data-monitoring device, neither the patient nor the physician will be able to adjust a function of the active implantable medical device by the data connection via the smartphone. Rather, any manipulation will only take place on the level of the data obtained by the sensing unit of the active implantable medical device.

It is apparent from the precedingly explained embodiment that no direct communication between the data transfer interface and the remote data-monitoring device is necessary in order to achieve a data transfer from the implantable medical device to the remote data-monitoring device. Rather, an indirect data communication between the data transfer interface and the remote data-monitoring device, e.g., via a smartphone, a Wi-Fi device, a mesh device (such as an mesh router or a mesh gateway) or any other wireless data communication device particularly suited for mediating a data communication between the data transfer interface and the remote data-monitoring device (i.e., a wireless communication mediating device), can be established in an embodiment. For this purpose, the distance between the wireless communication mediating device and the data transfer interface may be significantly shorter than a distance between the wireless communication mediating device and the remote data-monitoring device.

In an embodiment, the active implantable medical device is a device chosen from the group consisting of a cardiac rhythm monitor (loop monitor), a vital data monitor, an implantable pulse generator (IPG), an implantable cardioverter/defibrillator (ICD), a cardiac resynchronization device (CRT-P), a cardiac resynchronization device with the defibrillator (CRT-D), a glucose monitor, and a neurostimulator. A particularly appropriate vital data monitor is a monitor that monitors an intracardiac electrogram, an electrocardiogram, a breathing of the patient, a temperature of the patient, a coughing of the patient, an electroencephalogram of the patient, a blood pressure, a pulmonary artery pressure, a physical patient activity and/or other data that reflects the vitality of the patient.

In an embodiment, the active implantable medical device is a cardiac rhythm monitor comprising an automatic implantation recognition function. Such an automatic implantation recognition function enables the active implantable medical device to automatically detect its implantation (e.g., by an impedance measurement). In this embodiment, the active implantable medical device is configured to automatically activate the sensing of the at least one diagnostic parameter and the transfer of the sensed data to the remote data-monitoring device upon successful implantation recognition. Thus, as long as the active implantable medical device is not implanted, it will not waste any energy in attempts of measuring the at least one diagnostic parameter (which cannot be measured prior to implantation of the active implantable medical device). Rather, the relevant sensing and data transferring functionality will only be activated upon implantation of the active implantable medical device. This optimizes the power consumption of the active implantable medical device. Furthermore, the automatic implantation recognition facilitates handling of the active implantable medical device and reduces the risk of an incorrect operation (e.g., forgetting a manual activation of the data sensing and data transferring functions).

In an embodiment, the active implantable medical device comprises a data storage (or memory device) for storing the sensed data prior to transferring it to the remote data-monitoring device. Such a data storage can store the sensed data for a pre-defineable period of time, e.g., for one day, prior to starting a data transfer. In doing so, the energy required for the data transmission can be further optimized since in some embodiments the most power will be consumed for establishing a connection between the data transfer interface and (directly or indirectly) the remote data-monitoring device, wherein the data transfer itself requires considerably less power than establishing a connection.

In an embodiment, the data storage (memory unit) is a static random access memory (RAM) or a flash memory device. These types of storage devices require only very few energy for proper functioning and safely storing data until it is transferred by the data transfer interface.

In an embodiment, the diagnostic parameter is chosen from the group consisting of an intracardiac electrogram (EGM), an electrocardiogram (ECG), an electroencephalogram (EEG), a pressure curve, a breathing curve, an activity curve, and a temperature curve. These diagnostic parameters are particularly appropriate to evaluate specific physiologic or organic function of the human or animal patient.

In an embodiment, the at least one configuration parameter serves for modulating the sensitivity of detection of the at least one diagnostic parameter in the data received from the active implantable medical device. Alternatively or additionally, the at least one configuration parameter serves for modulating a specificity of detection of the at least one diagnostic parameter in the data received from the active implantable medical device. Thus, by amending the configuration parameter, it is possible to adjust the sensitivity and/or the specificity according to the needs, e.g., according to the medical question underlying the data evaluation. While prior art techniques teach to adjust the physical settings within the active implantable medical device, the presently claimed implant arrangement enables a purely virtual reprogramming of the active implantable medical device by manipulating the already obtained data and thus by post-measuring activities.

In an embodiment, the at least one configuration parameter serves for modulating a continuous evaluation of a physiologic curve representing the at least one diagnostic parameter. Appropriate examples for such a physiologic curve are an EGM, an ECG, an EEG, a pressure curve, a breathing curve, an activity curve and/or a temperature curve. Basically any physiologic parameter that is monitored over time so as to obtain a course of this physiologic parameter is appropriate to represent the physiologic curve. When an EGM or ECG is continuously evaluated, in particular P waves and R waves are detected within the EGM or ECG curve. Amending the at least one configuration parameter then serves for modulating the parameters of P-wave and R-wave detection within the EGM or ECG curve. This enables a very fine-tuned evaluation of the already obtained data and thus a very effective extraction of physiologic information contained in the obtained data.

In an embodiment, the at least one configuration parameter serves for modulating a sensing threshold of the at least one diagnostic parameter in the data received from the active implantable medical device. While the data is, according to an embodiment, sensed with the lowest possible threshold, such sensing threshold can then be set to a higher value in order to only evaluate highly relevant physiologic events, i.e., events that have exceeded a higher sensing threshold. Of course, the configuration parameter can be adjusted once again to lower the sensing threshold afterwards since all relevant information is present in the sensed data. The configuration parameter merely serves for allowing a more focused data evaluation that is tailored to the specific needs of the physician or medical staff performing the data analysis or evaluation.

In an embodiment, the at least one configuration parameter can be chosen from a predefined or predefineable set of configuration parameters. In this context, the configuration parameter is assigned to a type of diagnostics to be applied to a patient to whom the active implantable medical device is implanted. To give an example, a physician can choose a specific type of diagnostics to be applied to the patient. This choice can be made on a graphical user interface of the implant arrangement. This type of diagnostics is connected to one configuration parameter or a set of configuration parameters from the predefined set of configuration parameters. Then, these specific configuration parameters that are linked to the diagnostics to be applied will be automatically applied onto the data obtained by the remote data-monitoring device. Then, the data will be evaluated in a manner which is particularly appropriate for the underlying diagnostics. Consequently, the result of this data analysis is also tailored to the applied diagnostics and will thus be of high physiologic relevance for the underlying question.

To give some more specific examples for this embodiment, a set of configuration parameters can be defined for syncope diagnostics and a different set of configuration parameters can be defined for atrial fibrillation diagnostics. Furthermore, different sets of configuration parameters can be defined for different types of other specific diagnostics, either cardiac diagnostics or other diagnostics. Then, by simply choosing the diagnostics to be applied to the patient, the assigned set of configuration parameters will be applied onto the data and will deliver the relevant result for this type of diagnostics. At the same time, this result may not be that relevant for another type of diagnostics. However, if the other type of diagnostics is performed using a different set of configuration parameters, once again a relevant result for this other type of diagnostics will be delivered.

In an aspect, the present invention relates to a method of post-processing data on at least one diagnostic parameter of a human or animal patient. This method is performed on or with an implant arrangement, in particular an implant arrangement according to the preceding explanations. The method comprises the steps explained in the following.

In a first step, at least one diagnostic parameter of a human or animal patient is sensed with a sensing unit of an active implantable medical device. This active implantable medical device forms part of the implant arrangement.

In another method step, data on the at least one diagnostic parameter sensed by the sensing unit is transferred to a remote data-monitoring device. This remote data-monitoring device also forms part of the implant arrangement. The data transfer is achieved via a data transfer interface of the active implantable medical device.

In a further method step, the remote data-monitoring device applies at least one post-processing function on the data received from the active implantable medical device. As a result, post-processed data is obtained.

In a further method step, a user input is received by the remote data-monitoring device.

This user input serves in another method step for adjusting at least one configuration parameter of the post-processing function. Depending on the received user input, different adjustments of the at least one configuration parameter will be applied.

In a further method step, an effect of the post-processing function on the data received from the active implantable medical device is altered by the at least one configuration parameter. This effect can be, e.g., a sensitivity of detection, a specificity of detection and/or a kind of evaluation of a continuous signal comprising recurring signal features (like an EGM or ECG).

This method represents a virtual programming of the active implantable medical device without physically altering the state of the active implantable medical device. Rather, the method is purely based on the manipulation of the data already obtained by the active implantable medical device and by analyzing and evaluating this data according to the specific needs of the underlying question.

In an embodiment, the data transfer interface does not allow receiving data sent to the active implantable medical device. In a further embodiment of the method, the whole active implantable medical device does not allow receiving any data sent to the active implantable medical device. These embodiments significantly increase the cyber security of the implant arrangement used for carrying out the method. Reference is made in this respect also to the explanations given above with respect to cyber security aspects of the presently claimed implant arrangement.

All embodiments of the implant arrangement can be combined in any desired manner and can be transferred either individually or in any arbitrary combination to the method. Likewise, all embodiments of the method can be combined in any desired manner and can be transferred either individually or in any arbitrary combination to the implant arrangement.

Further details of aspects of the present invention will be explained referring to exemplary embodiments and accompanying Figures. In the Figures:
- Fig. 1: shows an first implant arrangement known from prior art;
- Fig. 2: shows a second implant arrangement also known from prior art;
- Fig. 3: shows an embodiment of a novel implant arrangement;
- Fig. 4: is a schematic depiction of an example of a virtual programming of an active implantable medical device; and
- Fig. 5: is a schematic flowchart of an embodiment of a method of post-processing data.

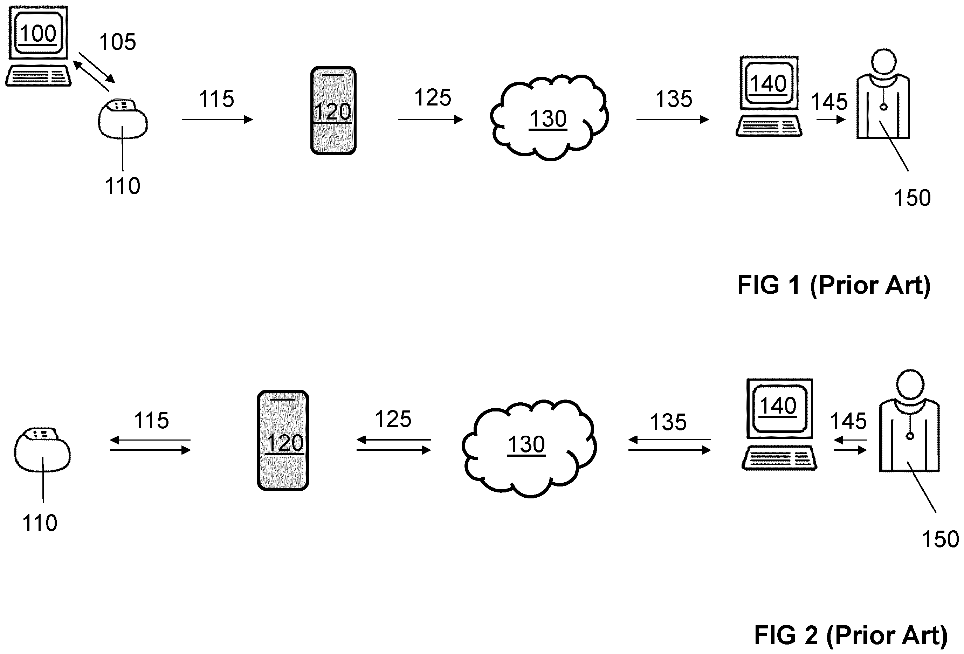

Fig. 1 shows an implant arrangement known from prior art comprising a cardiac rhythm monitor 110 that gathers diagnostic data from a human patient and transfers this data via a Bluetooth Low Energy (BLE) connection 115 to a smartphone 120 of the patient. The BLE connection serves only for transferring data from the cardiac rhythm monitor 110 to the smartphone 120, not in the opposite direction. A manipulation of settings of the cardiac rhythm monitor 110 via this BLE connection 115 is impossible. Consequently, the requirements with respect to cyber security of the BLE connection 115 are relatively low and securing this BLE connection 115 with BLE standard methods is possible.

The sensed diagnostic data are then transferred via a mobile connection 125 to a cloud-based remote monitoring system 130. A physician 150 can get access 145 via a regular personal computer 140 and a secured data connection 135 between the cloud-based remote monitoring system 130 and the personal computer 140.

In this prior art example, the data is only evaluated by the physician 150 without making it possible for the physician 150 to take any influence on the data stored in the remote monitoring system 130.

If any parameters for detecting the diagnostic data within the cardiac rhythm monitor 110 are to be changed, a regular programming device 100 is used that communicates with the cardiac rhythm monitor 110 via a secured bidirectional connection 105. This requires the presence of the patient carrying the cardiac rhythm monitor 110. Remote access to the cardiac rhythm monitor 110 via the BLE connection 115, the mobile connection 125 and/or the secured data connection 135 is not possible in this example.

Fig. 2 shows another implant arrangement known from prior art that allows a remote access via the previously mentioned connections. In this and all following Figures, similar elements will be denoted with the same numeral reference.

In the prior art example shown in Fig. 2, the physician 150 can adjust settings of the cardiac rhythm monitor 110 from his or her personal computer 140 via the secured data connection 135, the mobile connection 125 and the BLE connection 115. Thus, all these connections enable a bidirectional data transfer and thus enable the physician 150 to amend the diagnostic functions of the cardiac rhythm monitor 110.

In this context, it is rather simple to keep the secured data connection 135 between the personal computer 140 and the cloud-based remote monitoring system 130 up-to-date since both the personal computer 140 and the cloud-based remote monitoring system 130 are typically professionally administered and maintained.

However, securing the mobile connection 125 between the cloud-based remote monitoring system 130 and the smartphone 120 of the patient is much more sophisticated. This mobile connection 125 depends on the software status of the smartphone 120 of the patient. Updates of the software of the smartphone 120 lie within the responsibility of the patient and depend on patches provided by the operating system manufacturer. The same holds true for the BLE connection 115 between the smartphone 120 and the cardiac rhythm monitor 110.

Securing the BLE connection 115 and the mobile connection 125 thus requires additional effort and thus represents a potential cyber security risk and/or an availability risk that may limit the lifetime of the cardiac rhythm monitor 110 (e.g., in case of a discontinuation of operating system updates by the smartphone manufacturer or the operating system manufacturer).

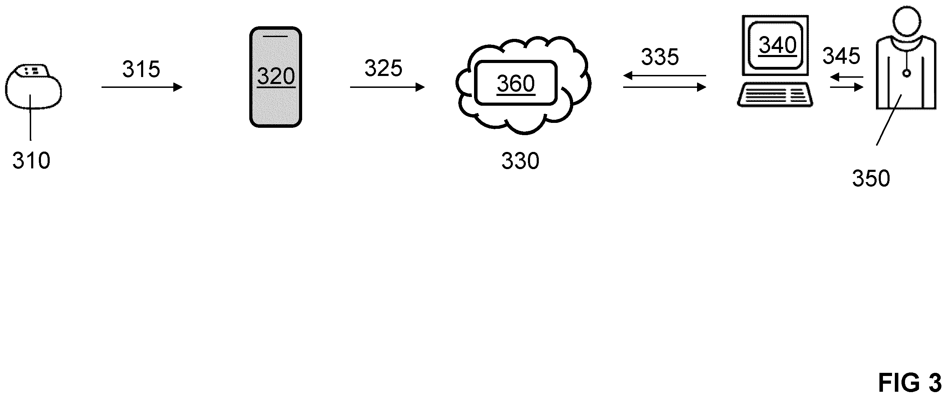

Fig. 3 shows an embodiment of an implant arrangement according to the presently claimed invention. A cardiac rhythm monitor 310 is connected via a BLE connection 315 with a smartphone 320 of a patient. This BLE connection 315 is established via a data transfer interface of the cardiac rhythm monitor 310. The smartphone 320 of the patient is typically located in relative proximity to the cardiac rhythm monitor 310 so that the BLE connection 315 only needs to bridge a rather short distance.

The smartphone 320 then further transfers the data received from the cardiac rhythm monitor 310 via a mobile connection 325 to a cloud-based remote monitoring system 330 serving as remote data-monitoring device. This cloud-based remote monitoring system 330 is connected via a secured data connection 335 with a personal computer 340 of a physician 350 who can gain access 345 to the data presented on the personal computer 340.

If the physician 350 desires to manipulate settings on the cardiac rhythm monitor 310, this will only be allowed in a virtual manner by manipulating the data provided to the cloud-based remote monitoring system 330 with the help of a configurable data post-processing device 360 forming part of the cloud-based remote monitoring system 330. Thus, the desired manipulations will only affect the sensed and already transferred data, not the settings of the cardiac rhythm monitor 310 itself. Consequently, it is no longer required to take care of additionally securing the BLE connection 315 and/or the mobile connection 325. These connections will not be used for transferring data towards the cardiac rhythm monitor 310, but only for transferring data away from the cardiac rhythm monitor 310 towards the cloud-based remote monitoring system 330.

The cardiac rhythm monitor 310 is configured such that its diagnostic parameters are designed for maximum sensitivity for all relevant monitoring applications like monitoring of a syncope, an atrial fibrillation monitoring, monitoring of a cryptogenic stroke, and/or monitoring of palpitations. In addition, the length of EGM or ECG recordings as well as memory and data transfer capacities are also designed to cover all physiological relevant monitoring applications.

The cardiac rhythm monitor 310 is also equipped with an automatic implantation recognition. This automatic implantation recognition enables an automatic activation of all diagnostic and data transfer functions of the cardiac rhythm monitor 310 after a successful recognition of an implantation of the cardiac rhythm monitor 310. Thus, no programming device is necessary for such activation. Therefore, the cardiac rhythm monitor 310 only comprises a data transfer interface for establishing the BLE connection 315 to the smartphone 320 of the patient. It does, however, not comprise any further data transfer interface so that it is physically impossible to influence the behavior of the cardiac rhythm monitor 310 with an external device. Consequently, the cardiac rhythm monitor 310 provides a high cyber security and completely avoids the risk of any cyber-attacks through an external network onto the cardiac rhythm monitor 310.

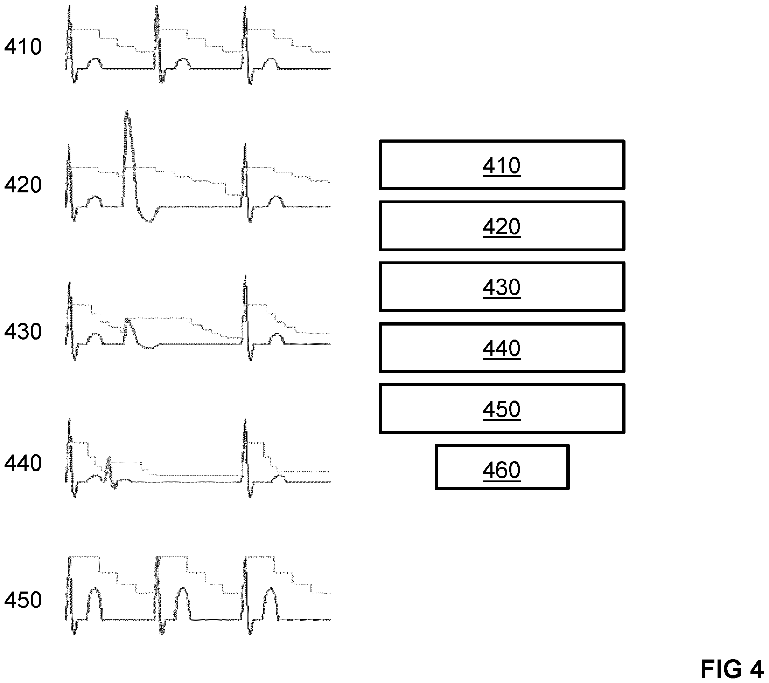

Fig. 4 exemplarily shows a diagnostic parameter that can be amended via virtual programming. For this purpose, a post-processing function is applied onto data representing this diagnostic parameter, wherein a configuration parameter is used for changing the effect of the post-processing function on the data relating to the diagnostic parameter. Consequently, different methods of applying sensing thresholds are possible that allow an appropriate cardiac rhythm detection.

This virtual programming shown in Fig. 4 is exemplarily applied to data provided by the cardiac rhythm monitor 310 shown in Fig. 3. This cardiac rhythm monitor 310 is operated in the most sensitive setting "Sense short intervals" 440. An intracardiac electrogram (EGM or IEGM) recorded with this setting is transferred by the cardiac rhythm monitor 310 via the BLE connection 315, the smartphone 320 of the patient and the mobile connection 325 to the cloud-based remote monitoring system 330. Depending on the virtual programming applied within the cloud-based remote monitoring system 330, different kinds of evaluation of the EGM is made possible.

To give some specific examples, a standard evaluation 410 may be applied featuring standard settings of increasing and decreasing sensing thresholds over a whole QRS complex in the EGM.

Alternatively, the setting "Sense after large premature ventricular contractions (PVCs)" 420 may be applied in which a different course of modifications of the sensing threshold is applied.

Alternatively, the setting "Sense small PVCs" 430 can be applied in which a lower sensing threshold is provided for detecting smaller cardiac signals.

Alternatively, the setting "Sense short intervals" 440, by which the data is originally recorded, may also be applied. Here, a significantly lower sensing threshold and faster sensing threshold decrease is implemented than in case of the standard evaluation 410.

Alternatively, the setting "T wave suppression" 450 may be applied in which the sensing threshold is chosen such high that T waves will no longer be detected in the EGM.

After having chosen one or more settings for evaluating the recorded EGM data, the user may choose to close the evaluation window on a graphical user interface by clicking on a close button 460.

Obviously, not only the exemplarily explained settings of a cardiac rhythm monitor may be virtually applied to the recorded EGM data, but virtually any other settings. The virtual programming is - as already extensively outlined above - not limited to cardiac rhythm monitors, but can be applied on all data sensed by any active implantable medical device.

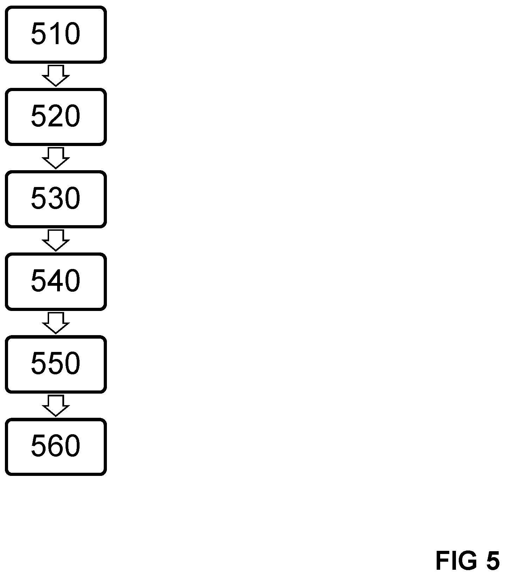

Fig. 5 schematically shows an embodiment of a method of post-processing data on at least one diagnostic parameter of a human or animal patient.

In a first step 510, at least one diagnostic parameter of a human or animal patient is sensed with a sensing unit of an active implantable medical device.

In a second step 520, data on the at least one diagnostic parameter sensed by the sensing unit is transferred to a remote data-monitoring device.

In a third step 530, the remote data-monitoring device applies at least one post-processing function on the data received from the active implantable medical device. As a result, post-processed data is obtained.

In a fourth step 540, a user input is received by the remote data-monitoring device.

This user input serves in fifth step 550 for adjusting at least one configuration parameter of the post-processing function.

In a sixth step 560, an effect of the post-processing function on the data received from the active implantable medical device is altered by the at least one configuration parameter.

## Claims

1. An implant arrangement comprising an active implantable medical device (310) and a remote data-monitoring device (330),
the active implantable medical device (310) comprising a sensing unit for sensing at least one diagnostic parameter of a human or animal patient and a data transfer interface for transferring data on the at least one diagnostic parameter sensed by the sensing unit to the remote data-monitoring device (330),
wherein the remote data-monitoring device (330) is configured to apply at least one post-processing function on the data received from the active implantable medical device (310),
**characterized in that** the remote data-monitoring device (330) is further configured to allow an adjustment of at least one configuration parameter of the post-processing function in dependence on a user input received by the remote data-monitoring device (330),
wherein the at least one configuration parameter alters an effect of the post-processing function on the data received from the active implantable medical device (310).

2. The implant arrangement according to claim 1, wherein the sensing unit is configured to sense the at least one diagnostic parameter with the lowest possible sensing threshold.

3. The implant arrangement according to claim 1 or 2, wherein the data transfer interface is configured to allow exclusively a data transfer away from the active implantable medical device (310), but not towards the active implantable medical device (310).

4. The implant arrangement according to any of the preceding claims, wherein the active implantable medical device (310) is configured to allow exclusively a data transfer away from the active implantable medical device (310), but not towards the active implantable medical device (310).

5. The implant arrangement according to any of the preceding claims, wherein the data transfer interface is the only data transfer interface of the active implantable medical device (310).

6. The implant arrangement according to any of the preceding claims, wherein the active implantable medical device (310) is a device chosen from the group consisting of a cardiac rhythm monitor, a vital data monitor, an implantable pulse generator, an implantable cardioverter/defibrillator, a cardiac resynchronization device, a cardiac resynchronization device with a defibrillator, a glucose monitor, and a neurostimulator.

7. The implant arrangement according to any of the preceding claims, wherein the active implantable medical device (310) is a cardiac rhythm monitor comprising an automatic implantation recognition function, wherein the active implantable medical device (310) is configured to automatically activate sensing the at least one diagnostic parameter and transferring the sensed data to the remote data-monitoring device (330) upon successful implantation recognition.

8. The implant arrangement according to any of the preceding claims, wherein the active implantable medical device (310) comprises a data storage for storing the sensed data prior to transferring it to the remote data-monitoring device (330).

9. The implant arrangement according to any of the preceding claims, wherein the diagnostic parameter is chosen from the group consisting of an electrocardiogram, an electroencephalogram, a pressure curve, a breathing curve, an activity curve, and a temperature curve.

10. The implant arrangement according to any of the preceding claims, wherein the at least one configuration parameter serves for modulating at least one of a sensitivity of detection of the at least one diagnostic parameter in the data received from the active implantable medical device (310) and a specificity of detection of the at least one diagnostic parameter in the data received from the active implantable medical device (310).

11. The implant arrangement according to any of the preceding claims, wherein the at least one configuration parameter serves for modulating a continuous evaluation of a physiologic curve representing the at least one diagnostic parameter.

12. The implant arrangement according to any of the preceding claims, wherein the at least one configuration parameter serves for modulating a sensing threshold of the at least one diagnostic parameter in the data received from the active implantable medical device (310).

13. The implant arrangement according to any of the preceding claims, wherein the at least one configuration parameter can be chosen from a predefined set of configuration parameters and is assigned to a type of diagnostics to be applied to a patient to whom the active implantable medical device (310) is implanted.

14. A method of post-processing data on at least one diagnostic parameter of a human or animal patient with an implant arrangement, in particular an implant arrangement according to any of the preceding claims, the method comprising the steps of:
a) sensing (510) at least one diagnostic parameter of a human or animal patient with a sensing unit of an active implantable medical device (310);
b) transferring (520) data on the at least one diagnostic parameter sensed by the sensing unit to a remote data-monitoring device (330) via a data transfer interface of the active implantable medical device;
c) applying (530), with the remote data-monitoring device (330), at least one post-processing function on the data received from the active implantable medical device (310), thus obtaining post-processed data;
d) receiving (540), by the remote data-monitoring device (330), a user input; the method **characterized by**:
e) adjusting (550) at least one configuration parameter of the post-processing function in dependence on the received user input; and
f) altering (560), by the at least one configuration parameter, an effect of the post-processing function on the data received from the active implantable medical device (310).

15. The method of claim 14, wherein the data transfer interface does not allow receiving data sent to the active implantable medical device (310).

## Patentansprüche

1. Eine Implantat-Anordnung, die eine aktive implantierbare medizinische Vorrichtung (310) und eine Datenfernüberwachungsvorrichtung (330) umfasst,
wobei die aktive implantierbare medizinische Vorrichtung (310) eine Erfassungseinheit zum Erfassen mindestens eines Diagnoseparameters eines menschlichen oder tierischen Patienten und eine Datenübertragungsschnittstelle zum Übertragen von Daten zu dem mindestens einen Diagnoseparameter, der von der Erfassungseinheit erfasst wird, an die Datenfernüberwachungsvorrichtung (330) umfasst,
wobei die Datenfernüberwachungsvorrichtung (330) so konfiguriert ist, dass sie mindestens eine Nachbearbeitungsfunktion auf die von der aktiven implantierbaren medizinischen Vorrichtung (310) empfangenen Daten anwendet,
**dadurch gekennzeichnet, dass** die Datenfernüberwachungsvorrichtung (330) ferner so konfiguriert ist, dass sie eine Anpassung mindestens eines Konfigurationsparameters der Nachbearbeitungsfunktion in Abhängigkeit von einer durch die Datenfernüberwachungsvorrichtung (330) empfangenen Benutzereingabe ermöglicht,
wobei der mindestens eine Konfigurationsparameter eine Wirkung der Nachbearbeitungsfunktion auf die von der aktiven implantierbaren medizinischen Vorrichtung (310) empfangenen Daten verändert.

2. Implantat-Anordnung nach Anspruch 1, wobei die Erfassungseinheit so konfiguriert ist, dass sie den mindestens einen Diagnoseparameter mit der niedrigstmöglichen Erfassungsschwelle erfasst.

3. Implantat-Anordnung nach Anspruch 1 oder 2, wobei die Datenübertragungsschnittstelle so konfiguriert ist, dass sie ausschließlich eine Datenübertragung von der aktiven implantierbaren medizinischen Vorrichtung (310) weg, aber nicht zu der aktiven implantierbaren medizinischen Vorrichtung (310) hin ermöglicht.

4. Implantat-Anordnung nach einem der vorhergehenden Ansprüche, wobei die aktive implantierbare medizinische Vorrichtung (310) so konfiguriert ist, dass sie ausschließlich eine Datenübertragung von der aktiven implantierbaren medizinischen Vorrichtung (310) weg, aber nicht zu der aktiven implantierbaren medizinischen Vorrichtung (310) hin erlaubt.

5. Implantat-Anordnung nach einem der vorhergehenden Ansprüche, wobei die Datenübertragungsschnittstelle die einzige Datenübertragungsschnittstelle der aktiven implantierbaren medizinischen Vorrichtung (310) ist.

6. Implantat-Anordnung nach einem der vorhergehenden Ansprüche, wobei die aktive implantierbare medizinische Vorrichtung (310) eine Vorrichtung ist, die aus der Gruppe ausgewählt ist, die aus einem Herzrhythmusmonitor, einem Vitaldatenmonitor, einem implantierbaren Pulsgenerator, einem implantierbaren Kardioverter/Defibrillator, einer kardialen Resynchronisationsvorrichtung, einer kardialen Resynchronisationsvorrichtung mit einem Defibrillator, einem Glukosemonitor und einem Neurostimulator besteht.

7. Implantat-Anordnung nach einem der vorhergehenden Ansprüche, wobei die aktive implantierbare medizinische Vorrichtung (310) ein Herzrhythmusmonitor ist, der eine automatische Implantationserkennungsfunktion umfasst, wobei die aktive implantierbare medizinische Vorrichtung (310) so konfiguriert ist, dass sie bei erfolgreicher Implantationserkennung automatisch die Erfassung des mindestens einen Diagnoseparameters aktiviert und die erfassten Daten an die Datenfernüberwachungsvorrichtung (330) überträgt.

8. Implantat-Anordnung nach einem der vorhergehenden Ansprüche, wobei die aktive implantierbare medizinische Vorrichtung (310) einen Datenspeicher zum Speichern der erfassten Daten vor ihrer Übertragung an die Datenfernüberwachungsvorrichtung (330) umfasst.

9. Implantat-Anordnung nach einem der vorhergehenden Ansprüche, wobei der Diagnoseparameter aus der Gruppe bestehend aus einem Elektrokardiogramm, einem Elektroenzephalogramm, einer Druckkurve, einer Atemkurve, einer Aktivitätskurve und einer Temperaturkurve ausgewählt wird.

10. Implantat-Anordnung nach einem der vorhergehenden Ansprüche, wobei der mindestens eine Konfigurationsparameter dazu dient, mindestens eine Sensitivität der Detektion des mindestens einen Diagnoseparameters in den von der aktiven implantierbaren medizinischen Vorrichtung (310) empfangenen Daten und eine Spezifität der Detektion des mindestens einen Diagnoseparameters in den von der aktiven implantierbaren medizinischen Vorrichtung (310) empfangenen Daten zu modulieren.

11. Implantat-Anordnung nach einem der vorhergehenden Ansprüche, wobei der mindestens eine Konfigurationsparameter zur Modulation einer kontinuierlichen Bewertung einer physiologischen Kurve dient, die den mindestens einen Diagnoseparameter darstellt.

12. Implantat-Anordnung nach einem der vorhergehenden Ansprüche, wobei der mindestens eine Konfigurationsparameter zur Modulation einer Erfassungsschwelle des mindestens einen Diagnoseparameters in den von der aktiven implantierbaren medizinischen Vorrichtung (310) empfangenen Daten dient.

13. Implantat-Anordnung nach einem der vorhergehenden Ansprüche, wobei der mindestens eine Konfigurationsparameter aus einem vordefinierten Satz von Konfigurationsparametern auswählbar und einer Art von Diagnostik zugeordnet ist, die bei einem Patienten anzuwenden ist, dem die aktive implantierbare medizinische Vorrichtung (310) implantiert wird/ist.

14. Verfahren zur Nachbearbeitung von Daten zu mindestens einem Diagnoseparameter eines menschlichen oder tierischen Patienten mit einer Implantat-Anordnung, insbesondere einer Implantat-Anordnung nach einem der vorherehenden Ansprüche, wobei das Verfahren die folgenden Schritte umfasst:
a) Erfassen (510) mindestens eines Diagnoseparameters eines menschlichen oder tierischen Patienten mit einer Erfassungseinheit einer aktiven implantierbaren medizinischen Vorrichtung (310);
b) Übertragen (520) von Daten zu dem mindestens einen von der Erfassungseinheit erfassten Diagnoseparameter an eine Datenfernüberwachungsvorrichtung (330) über eine Datenübertragungsschnittstelle der aktiven implantierbaren medizinischen Vorrichtung;
c) Anwendung (530) mindestens einer Nachbearbeitungsfunktion auf die von der aktiven implantierbaren medizinischen Vorrichtung (310) empfangenen Daten mit der Datenfernüberwachungsvorrichtung (330), um so nachbearbeitete Daten zu erhalten;
d) Empfangen (540) einer Benutzereingabe durch die Datenfernüberwachungsvorrichtung (330); wobei das Verfahren **gekennzeichnet ist durch**:
e) Anpassen (550) mindestens eines Konfigurationsparameters der Nachbearbeitungsfunktion in Abhängigkeit von der empfangenen Benutzereingabe; und
f) Ändern (560) einer Wirkung der Nachbearbeitungsfunktion auf die von der aktiven implantierbaren medizinischen Vorrichtung (310) empfangenen Daten durch den mindestens einen Konfigurationsparameter.

15. Verfahren nach Anspruch 14, wobei die Datenübertragungsschnittstelle den Empfang von Daten, die an die aktive implantierbare medizinische Vorrichtung (310) gesendet werden, nicht zulässt.

## Revendications

1. Un dispositif d'implantation comprenant un dispositif médical implantable actif (310) et un dispositif de surveillance des données à distance (330),
le dispositif médical implantable actif (310) comprend une unité de détection pour détecter au moins un paramètre de diagnostic d'un patient humain ou animal et une interface de transfert de données pour transférer des données sur le au moins un paramètre de diagnostic détecté par l'unité de détection au dispositif de surveillance des données à distance (330),
dans lequel le dispositif de surveillance des données à distance (330) est configuré pour appliquer au moins une fonction de post-traitement aux données reçues du dispositif médical implantable actif (310),
**caractérisé en ce que** le dispositif de surveillance des données à distance (330) est en outre configuré pour permettre un ajustement d'au moins un paramètre de configuration de la fonction de post-traitement en fonction d'une entrée utilisateur reçue par le dispositif de surveillance des données à distance (330),
dans lequel l'au moins un paramètre de configuration modifie un effet de la fonction de post-traitement sur les données reçues du dispositif médical implantable actif (310).

2. L'implant selon la revendication 1, dans lequel l'unité de détection est configurée pour détecter au moins un paramètre de diagnostic avec le seuil de détection le plus bas possible.

3. Le dispositif d'implantation selon la revendication 1 ou 2, dans lequel l'interface de transfert de données est configurée pour permettre exclusivement un transfert de données à partir du dispositif médical implantable actif (310), mais pas en direction du dispositif médical implantable actif (310).

4. Le dispositif d'implantation selon l'une des revendications précédentes, dans lequel le dispositif médical implantable actif (310) est configuré pour permettre exclusivement un transfert de données à partir du dispositif médical implantable actif (310), mais pas en direction du dispositif médical implantable actif (310).

5. Le dispositif d'implantation selon l'une des revendications précédentes, dans lequel l'interface de transfert de données est la seule interface de transfert de données du dispositif médical implantable actif (310).

6. Le dispositif d'implantation selon l'une des revendications précédentes, dans lequel le dispositif médical implantable actif (310) est un dispositif choisi dans le groupe constitué d'un moniteur de rythme cardiaque, d'un moniteur de données vitales, d'un générateur d'impulsions implantable, d'un cardioverteur/défibrillateur implantable, d'un dispositif de resynchronisation cardiaque, d'un dispositif de resynchronisation cardiaque avec défibrillateur, d'un moniteur de glucose, et d'un neurostimulateur.

7. Dispositif d'implantation selon l'une des revendications précédentes, dans lequel le dispositif médical implantable actif (310) est un moniteur de rythme cardiaque comprenant une fonction de reconnaissance automatique de l'implantation, dans lequel le dispositif médical implantable actif (310) est configuré pour activer automatiquement la détection d'au moins un paramètre de diagnostic et le transfert des données détectées vers le dispositif de surveillance des données à distance (330) en cas de reconnaissance réussie de l'implantation.

8. Le dispositif d'implantation selon l'une des revendications précédentes, dans lequel le dispositif médical implantable actif (310) comprend une mémoire de données pour stocker les données détectées avant de les transférer au dispositif de surveillance des données à distance (330).

9. Dispositif d'implantation selon l'une des revendications précédentes, dans lequel le paramètre de diagnostic est choisi dans le groupe constitué d'un électrocardiogramme, d'un électroencéphalogramme, d'une courbe de pression, d'une courbe de respiration, d'une courbe d'activité et d'une courbe de température.

10. Le dispositif d'implantation selon l'une quelconque des revendications précédentes, dans lequel l'au moins un paramètre de configuration sert à moduler au moins l'une d'une sensibilité de détection de l'au moins un paramètre de diagnostic dans les données reçues du dispositif médical implantable actif (310) et une spécificité de détection de l'au moins un paramètre de diagnostic dans les données reçues du dispositif médical implantable actif (310).

11. L'implant selon l'une des revendications précédentes, dans lequel l'au moins un paramètre de configuration sert à moduler une évaluation continue d'une courbe physiologique représentant l'au moins un paramètre de diagnostic.

12. Le dispositif d'implantation selon l'une des revendications précédentes, dans lequel l'au moins un paramètre de configuration sert à moduler un seuil de détection de l'au moins un paramètre de diagnostic dans les données reçues du dispositif médical implantable actif (310).

13. Le dispositif d'implantation selon l'une des revendications précédentes, dans lequel l'au moins un paramètre de configuration peut être choisi dans un ensemble prédéfini de paramètres de configuration et est affecté à un type de diagnostic à appliquer à un patient sur lequel le dispositif médical implantable actif (310) est implanté.

14. Procédé de post-traitement des données relatives à au moins un paramètre de diagnostic d'un patient humain ou animal porteur d'un dispositif d'implantation, en particulier d'un dispositif d'implantation selon l'une quelconque des revendications précédentes, le procédé comprenant les étapes suivantes :
a) détecter (510) au moins un paramètre de diagnostic d'un patient humain ou animal à l'aide d'une unité de détection d'un dispositif médical implantable actif (310) ;
b) transférer (520) les données relatives à l'au moins un paramètre de diagnostic détecté par l'unité de détection à un dispositif de surveillance des données à distance (330) via une interface de transfert de données du dispositif médical implantable actif ;
c) appliquer (530), avec le dispositif de surveillance des données à distance (330), au moins une fonction de post-traitement sur les données reçues du dispositif médical implantable actif (310), obtenant ainsi des données post-traitées ;
d) recevoir (540), par le dispositif de surveillance des données à distance (330), une entrée de l'utilisateur ; le procédé est **caractérisé par** :
e) ajuster (550) au moins un paramètre de configuration de la fonction de post-traitement en fonction de l'entrée utilisateur reçue ; et
f) modifier (560), au moyen d'au moins un paramètre de configuration, un effet de la fonction de post-traitement sur les données reçues du dispositif médical implantable actif (310).

15. Le procédé de la revendication 14, dans laquelle l'interface de transfert de données ne permet pas de recevoir des données envoyées au dispositif médical implantable actif (310).
